# EUROPÄISCHE PATENTSCHRIFT

(11) **EP 0 574 795 B1**
(45) Veröffentlichungstag und Bekanntmachung des Hinweises auf die Patenterteilung: **11.09.1996**
(21) Anmeldenummer: 93109151.6
(22) Anmeldetag: 08.06.1993
(51) Int. Cl.: C07D 239/26, C07D 239/06

(54) **Verfahren zur Herstellung von Pyrimidinen**
Process for the preparation of pyrimidines
Procédé pour la préparation de pyrimidines

(30) Priorität: 17.06.1992 DE 4219789
(43) Veröffentlichungstag der Anmeldung: 22.12.1993
(73) Patentinhaber: BASF Aktiengesellschaft, 67063 Ludwigshafen (DE)
(72) Erfinder: Ebel, Klaus, Dr., W-6700 Ludwigshafen (DE); Schroeder, Juergen, Dr., W-6806 Viernheim (DE)

(56) Entgegenhaltungen:
- EP-A- 0 192 299
- DE-A- 4 024 259
- PATENT ABSTRACTS OF JAPAN vol. 14, no. 310 (C-736)(4253) 4. Juli 1990 & JP-A-2104577 (KOEI CHEM CO LTD) 17 April 1990
- PATENT ABSTRACTS OF JAPAN vol. 14, no. 137 (C-702)(4080) 15. März 1990 & JP-A-2011577 (KOEI CHEM CO LTD) 16 January 1990

## Beschreibung

### Beschreibung

Die Erfindung betrifft ein neues Verfahren zur Herstellung von 2-substituierten Pyrimidinen durch kupfer-(II)-acetatkatalysierte Umsetzung von Carbonsäurenitrilen mit 1,3-Diaminopropanen zu den 2-substituierten 1,4,5,6-Tetrahydropyrimidinen und die anschließende Dehydrierung.

Die JP 02/104.577 beschreibt ein Verfahren zur Herstellung von Tetrahydropyrimidinen durch Umsetzung von Carbonsäurenitrilen mit 1,3-Diaminopropanen in Gegenwart von Katalysatoren wie z.B. Zinkacetat oder Kupfer-(II)-acetat.

EP-A-192 299 und JP-A-2-11577 beschreiben ein verfahren zur Herstellung von Pyrimidinen und DE-A-4 024 259 beschreibt ein Verfahren zur Herstellung von cyclischen Amidinen.

Aus Heterocycles 27, 1867 bis 1871 (1988) ist ein zweistufiges Verfahren zur Herstellung von 2-Alkylpyrimidinen bekannt, bei dem in der ersten Stufe Carbonsäuren mit einem großen Überschuß 1,3-Diaminopropan zu den entsprechenden 2-Alkyl-1,4,5,6-tetrahydropyrimidin umgesetzt werden und in der zweiten Stufe die gereinigten 2-Alkyl-1,4,5,6-tetra-hydropyrimidine bei 320 bis 350°C an Pd/Al₂O₃ zum 2-Alkylpyrimidin dehydriert werden.

Nachteilig bei dem Verfahren ist der Einsatz des teuren, empfindlichen Palladiumkatalysators bei der Dehydrierung.

Der vorliegenden Erfindung lag daher die Aufgabe zugrunde den obengenannten Nachteil abzuhelfen.

Demgemäß wurde ein neues Verfahren zur Herstellung von Pyrimidinen der allgemeinen Formel I 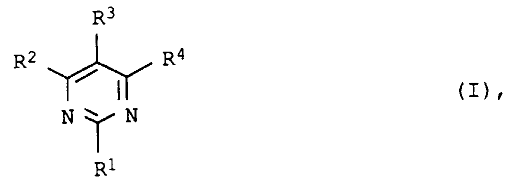 in der
- R¹: C₁- bis C₈-Alkyl, C₇- bis C₁₂-Aralkyl oder Phenyl und
- R²,R³,R⁴: unabhängig voneinander Wasserstoff, C₁- bis C₈-Alkyl, C₇- bis C₁₂-Aralkyl oder Phenyl bedeuten,
gefunden, welches dadurch gekennzeichnet ist, daß man Carbonsäurenitrile der allgemeinen Formel II

R¹-C≡N (II),

in der der Substituent R¹ die obengenannte Bedeutung hat, mit 1,3-Diaminopropanen der allgemeinen Formel III 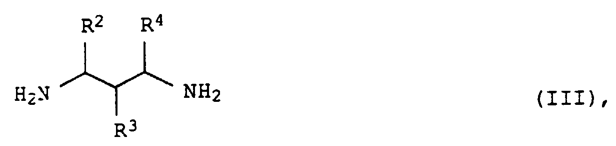 in der die Substituenten R² bis R⁴ die obengenannten Bedeutungen haben, in Gegenwart von Kupfer-(II)-acetat bei Temperaturen von 150 bis 250°C zu dem Tetrahydropyrimidin der allgemeinen Formel IV 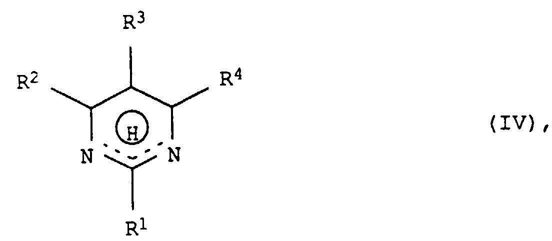 in der die Substituenten R¹ bis R⁴ die obengenannten Bedeutungen haben, umsetzt, und das Tetrahydropyrimidin der Formel IV mit Raney-Nickel bei Temperaturen von 150 bis 300°C zum Pyrimidin der Formel I dehydriert.

Das erfindungsgemäße Verfahren läßt sich in der Weise durchführen, daß man Carbonsäurenitrile der Formel II mit 1,3-Diaminopropanen der Formel III in stöchiometrischer Menge, im Über- oder Unterschuß, vorzugsweise im Molverhältnis 0,5 : 1 bis 1,5 : 1, bevorzugt 1 : 1 bis 1,1 : 1 kupfer-(II)-acetatkatalysiert zum 2-substituierten 1,4,5,6-Tetrahydropyrimidin der Formel IV umsetzt. Die Reaktion wird bei einer Temperatur von 150 bis 250°C durchgeführt, vorzugsweise bei 180 bis 220°C. Nach beendeter Reaktion werden nicht umgesetztes Carbonsäurenitil der Formel II und 1,3-Diaminopropan der Formel III abdestilliert, vorzugsweise bei Reaktiontemperatur und entsprechend vermindertem Druck. Anschließend wird bei Reaktionstemperatur mit Raney-Nickel versetzt und bei einer Temperatur von 150 bis 300°C, vorzugsweise bei 180 bis 250°C, zum 2-substituierten Pyrimidin der Formel I dehydriert. Die Dehydrierung wird vorzugsweise bei einem Druck durchgeführt, bei dem das 2-substituierte Pyrimidin der Formel I kontinuierlich abdestilliert.

Im Vergleich zu den bekannten Verfahren liefert das Verfahren nach der Erfindung auf einfacheren und wirtschaftlicherem Wege 2-substituierte Pyrimidine der Formel I.

Die Substituenten R¹, R², R³ und R⁴ der Verbindungen I, II, III und IV haben folgende Bedeutungen:
R¹,R²,R³,R⁴
- unabhängig voneiander
- C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl und iso-Octyl, bevorzugt C₁- bis C₄-Alkyl wie Methyl, Ethyl, n-Propyl, iso-Propyl, n-Butyl, iso-Butyl, sec.-Butyl und tert.-Butyl, besonders bevorzugt Methyl und Ethyl,
- C₇- bis C₁₂-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
- Phenyl,
R²,R³,R⁴
- Wasserstoff.

Synthetisch hergestellte Pyrimidine haben große Bedeutung als Zwischenprodukte für Pharmaka und für Wirkstoffe im Pflanzenschutz.

### Beispiele

### Beispiel 1

320 g (3,1 mol) Benzonitril und 5 g Kupfer(II)acetat werden in einem 1l-Rührkolben vorgelegt und bei 180 bis 190°C mit 222 g (3 mol) 1, 3-Propylendiamin versezt (ca. 2,5 Stunden). Nach weiteren 3 Stunden bei 180°C wird nicht umgesetztes Benzonitril und 1,3-Propylendiamin bei 180°C/60 mbar abdestilliert, mit einer Suspension aus 20 g Raney-Nickel in 20 ml Dimethylformamid versetzt und Wasser und Dimethylformamid bei 180°C/60 mbar abdestilliert. Bei 245°C beginnt die Dehydrierung; 2-Phenyl-pyrimidin wird dabei kontinuierlich abdestilliert (Sdp.: 191 bis 213°C/300 mbar). Die Rohausbeute beträgt 304 g (65 %) mit einer Reinheit von 87 %. Nach nochmaliger Destillation erhält man im Hauptlauf bei 120 bis 124°C/4 mbar 284 g (61 %) 2-Phenyl-pyrimidin mit einer Reinheit von 99 %.

### Beispiel 2

363 g (3,1 mol) Phenylacetonitril und 5 g Kupfer(II)acetat werden in einem 1l-Rührkolben vorgelegt und bei 180 bis 190°C mit 222 g (3 mol) 1,3-Propylendiamin versetzt (ca. 2,5 Stunden). Nach weiteren 3 Stunden bei 180°C wird nicht umgesetztes Phenylacetonitril und 1,3-Propylendiamin bei 180°C/60 mbar abdestilliert, mit einer Suspension aus 20 g Raney-Nickel in 20 ml Dimethylformamid versetzt und Wasser und Dimethylformamid bei 180°C/60 mbar abdestilliert. Bei 245°C beginnt die Dehydrierung; 2-Benzyl-pyrimidin wird dabei kontinuierlich abdestilliert (Sdp.: 176 bis 178°C/10 mbar). Die Ausbeute beträgt 317 g (62 %) mit einer Reinheit von 99 %.

## Patentansprüche

1. Verfahren zur Herstellung von Pyrimidinen der allgemeinen Formel I 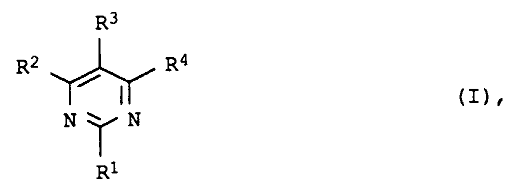 in der
R¹ C₁- bis C₈-Alkyl, C₇- bis C₁₂-Aralkyl oder Phenyl und
R²,R³,R⁴ unabhängig voneinander Wasserstoff, C₁- bis C₈-Alkyl, C₇- bis C₁₂-Aralkyl oder Phenyl bedeuten,
dadurch gekennzeichnet, daß man Carbonsäurenitrile der allgemeinen Formel II
R¹-C≡N (II),
in der der Substituent R¹ die obengenannte Bedeutung hat, mit 1,3-Diaminopropanen der allgemeinen Formel III 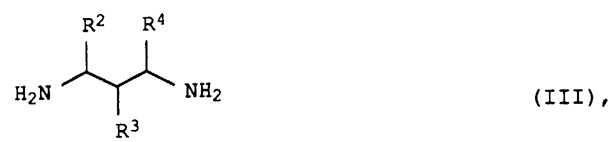 in der die Substituenten R² bis R⁴ die obengenannten Bedeutungen haben, in Gegenwart von Kupfer-(II)-acetat bei Temperaturen von 150 bis 250°C zu dem Tetrahydropyrimidin der allgemeinen Formel IV 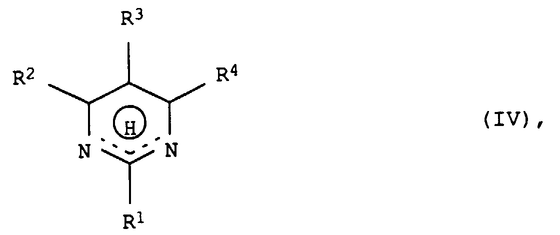 in der die Substituenten R¹ bis R⁴ die obengenannten Bedeutungen haben, umsetzt, und das Tetrahydropyrimidin der Formel IV mit Raney-Nickel bei Temperaturen von 150 bis 300°C zum Pyrimidin der Formel I dehydriert.

2. Verfahren nach Anspruch 1, dadurch gekennzeichnet, daß man die Reaktion als Eintopfsynthese durchführt.

3. Verfahren nach Anspruch 1 und 2, dadurch gekennzeichnet, daß das Pyrimidin der Formel I bei der Dehydrierung kontinuierlich abdestilliert wird.

## Claims

1. A process for preparing pyrimidines of the formula I 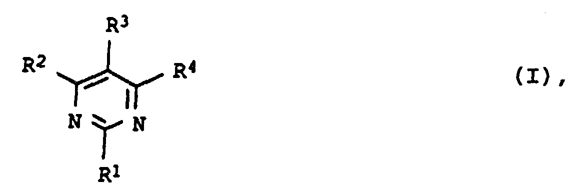 where
R¹ is C₁-C₈-alkyl, C₇-C₁₂-aralkyl or phenyl and
R², R³ and R⁴ are each, independently of one another, hydrogen, C₁-C₈-alkyl, C₇-C₁₂-aralkyl or phenyl,
which comprises reacting nitriles of the formula II
R¹-C≡N (II),
where R¹ has the abovementioned meaning, with 1,3-diaminopropanes of the formula III 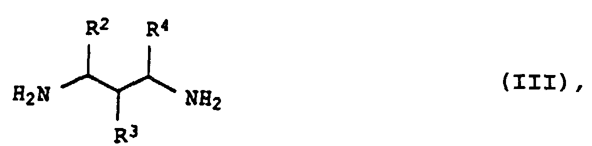 where R² to R⁴ have the abovementioned meanings, in the presence of copper(II) acetate at from 150 to 250°C to give the tetrahydropyrimidine of the formula IV 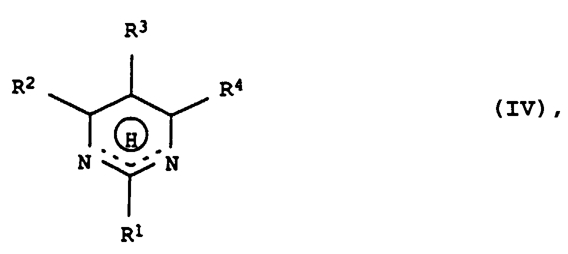 where R¹ to R⁴ have the abovementioned meanings, and dehydrogenating the tetrahydropyrimidine of the formula IV with Raney nickel at from 150 to 300°C to give the pyrimidine of the formula I.

2. A process as claimed in claim 1, wherein the reaction is carried out as a one-pot synthesis.

3. A process as claimed in claim 1 or 2, wherein the pyrimidine of the formula I is distilled out continuously during the dehydrogenation.

## Revendications

1. Procédé de préparation de pyrimidines de la formule générale I 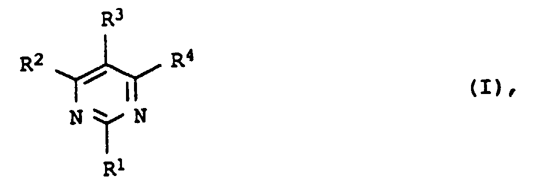 dans laquelle
R¹ représente un radical alkyle en C₁ à C₈, aralkyle en C₇ à C₁₂, ou phényle et
R², R³, R⁴ représentent, chacun indépendamment l'un de l'autre, un radical alkyle en C₁ à C₈, aralkyle en C₇ à C₁₂, ou phényle,
caractérisé en ce que l'on fait réagir des nitriles d' acides carboxyliques de la formule générale II
R¹-C≡N (II),
dans laquelle le substituant R¹ possède les significations qui lui ont été attribuées ci-dessus, avec des 1,3-diaminopropanes de la formule générale III 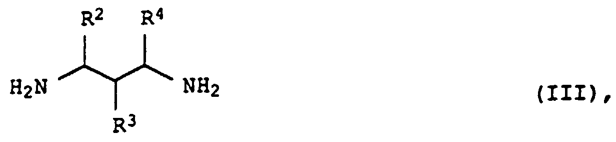 dans laquelle les substituants R² à R⁴ possèdent les significations qui leur ont été attribuées ci-dessus, en présence d'acétate de cuivre-(II) à des températures de 150 à 250°C, de manière à obtenir la tétrahydropyrimidine de la formule générale IV 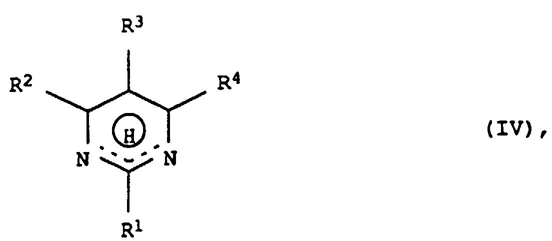 dans laquelle les substituants R¹ à R⁴ possèdent les significations qui leur ont été attribuées ci-dessus et on déshydrogène la tétrahydropyrimidine de la formule IV à l'aide de nickel de Raney à des températures de 150 à 300°C, de façon à obtenir la pyrimidine de la formule I.

2. Procédé suivant la revendication 1, caractérisé en ce que l'on entreprend la réaction sous forme de synthèse en un stade.

3. Procédé suivant les revendications 1 et 2, caractérisé en ce que l'on sépare continuellement la pyrimidine de la formule I par distillation au cours de la déshydrogénation.
